# EUROPEAN PATENT APPLICATION

(11) **EP 4 331 360 A1**
(43) Date of publication of application: **06.03.2024**
(21) Application number: 21727192.3
(22) Date of filing: 27.04.2021
(51) Int. Cl.: A01N 1/02, A61M 5/178

(54) **DEVICE AND METHOD FOR STORING AND DISPENSING A LIQUID SAMPLE**

(71) Applicant: Leartiker, S.Coop., 48270 Markina-Xemein (Bizkaia) (ES); Universidad de Navarra, 31009 Pamplona (Navarra) (ES)
(72) Inventor: ANDREU OLTRA, Enrique José, 31008 Pamplona (ES); GEREKA GOIENETXE, Ainitze, 48270 Markina-Xemein (ES); LARREATEGI MAKATZAGA, Pablo, 48270 Markina-Xemein (ES); PRÓSPER CARDOSO, Felipe Luis, 31008 Pamplona (ES); ZALDUA HUICI, Ane Miren, 48270 Markina-Xemein (ES)
(74) Representative: Igartua, Ismael
(86) International application number: PCT/ES2021/070284
(87) International publication number: WO 2022/229476

(57) **Abstract**

Device for storing and dispensing a liquid sample comprising a hollow body (8) comprising a chamber for storing the liquid sample and dispensing means for extracting the liquid sample from the chamber, wherein the device further comprises an inlet conduit (5) configured for introducing the liquid sample to the chamber, the inlet conduit (5) being configured to be closable in at least two axial positions (52, 51) in order to generate at least one pouch (53) in said inlet conduit for storing a pilot liquid sample. Method for storing and dispensing a liquid sample.

## Description

### TECHNICAL FIELD

The present invention relates to devices and methods for storing and dispensing liquid samples, preferably for preserving and dispensing biologic materials.

### PRIOR ART

Culturing, storing, preserving, and dispensing biologic material, for example cells, by injection or inoculation into a patient, is increasingly common in cell therapy treatment. Furthermore, the process of preserving the cells must not in itself damage or destroy the cells. The control of this process is needed in order to assure the viability of cells before the inoculation, thus, to assure the cells are biologically active materials when they are injected or inoculated to the patient.

One of the conventional methods for preserving cells is the cryopreservation usually involving a long-term storing in cryogenic conditions, mainly, at low or ultra low temperatures, for example, in liquid nitrogen. In current practice, cells are harvested, suspended in a storage solution, introduced in a cryoresistant vial and then frozen for preservation. When the cells are needed, they are then thawed, extracted from the vial and cultivated in a culture or inoculated to the patient with a syringe. The challenge to cells during cryopreservation is their ability to survive in intermediate zone of temperature (e.g., -15 to -60°C.) that cells must traverse twice, once during cooling and once during warming. During this process, the availability of counter samples or pilot samples of the stored and preserved cells is very helpful to control the cryogenization process and to test the viability of cells before the inoculation or injection of the cells into the patient.

US20060019233 A1 discloses a syringe for cryopreserving biological materials comprising a hollow body comprising a chamber for storing biological material and dispensing means for extracting and dispensing the liquid sample from the chamber.

### DISCLOSURE OF THE INVENTION

The object of the invention is to provide a device for storing and dispensing a liquid sample and a method for storing and dispensing a liquid sample carried out by means of the device, as defined in the claims.

One aspect of the invention relates to a device for storing and dispensing a liquid sample comprising a hollow body comprising a chamber for storing the liquid sample and dispensing means for extracting the liquid sample from the chamber. The device further comprises an inlet conduit configured for introducing the liquid sample into the chamber, being the inlet conduit to be closable in at least two axial positions in order to generate at least one pouch in said inlet conduit for storing a counter or pilot liquid sample.

Another aspect of the invention relates to a method for storing and dispensing a liquid sample carried out by means of the device which comprises the following steps:
- a first step of introducing the liquid sample to be stored into the chamber of the hollow body through the inlet conduit,
- a second step of generating in the inlet conduit at least one pouch for storing a pilot liquid sample,
- a third step of storing the liquid sample within the chamber and the at least one pouch, and
- a fourth step of dispensing the stored liquid sample.

The step of generating the at least one pouch for storing a pilot liquid sample in the inlet conduit comprises:
- a first step of isolating liquid sample within the inner conduit between a first axial position and a second axial position, and,
- a second step of closing the inlet conduit in the first axial position and the second axial position generating the at least one pouch

The device and the method of the invention allow generating counter or pilot samples within the same device that is filled with the liquid to be stored. This counter sample or pilot sample is generated attached to the device, thus, it is ensured that the counter sample and the liquid within the chamber are subjected to the same filling and storage conditions, and therefore the counter sample can be used as a control and testing sample during the storage and/or injection process without the need of manipulation of the liquid sample in the chamber. Furthermore, there is avoided having counter samples delocalized in the storage medium, for example, in the freezer cabinet, making to the user easier the use of the correct counter sample in the control and testing process. Furthermore, as the storage and dispensation are done with the same device, the risk of contamination of the liquid sample is minimized and the loss of liquid sample is avoided, having a better use of the stored liquid sample.

These and other advantages and features of the invention will become apparent in view of figures and detailed description of the invention.

### DESCRIPTION OF THE DRAWINGS

Figure 1 shows an exploded view of the different components of the device according to one embodiment of the invention.
Figure 2 shows a partial view of the device with the pouch generated in the inlet conduit of the device according to one embodiment of the invention.
Figure 3 shows a perspective view of the device according to one embodiment of the invention having a liquid sample stored in the chamber and ready for dispensing the liquid sample.

### DETAILED DISCLOSURE OF THE INVENTION

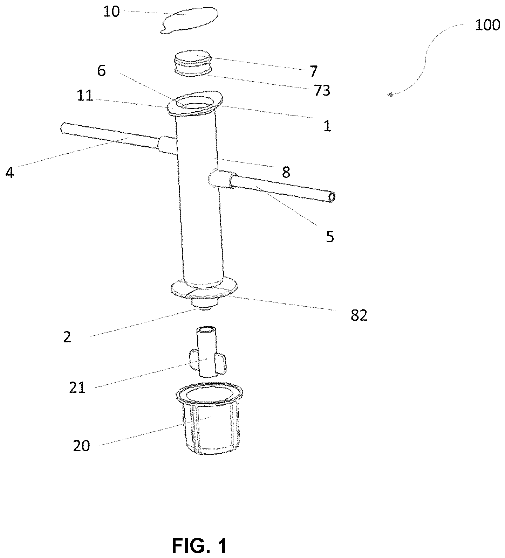

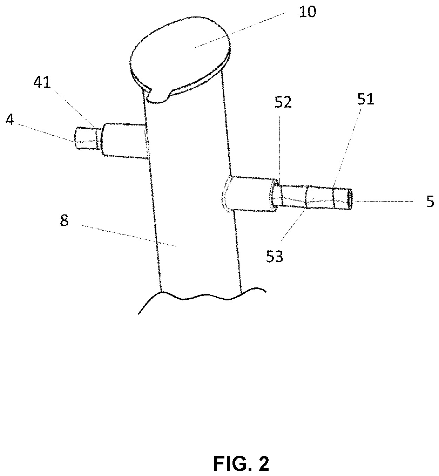

As shown in Figure 1 and 2, the device 100 of the invention for storing and dispensing a liquid sample 54 comprises a hollow body 8 comprising a chamber 6 for storing the liquid sample and dispensing means for extracting or dispensing the liquid sample from the chamber 6. The device further comprises an inlet conduit or a branch 5 in communication with the interior of the chamber 6. Preferably, the inlet conduit is located in the hollow body, more preferably being coupled to the hollow body 8 in a manner to provide a permanent, leak -proof sealed engagement. The inlet conduit 5 is configured for introducing the liquid sample into the chamber 6 through a chamber 6 and inlet conduit 5 connection opening (not shown in Figures). This inlet conduit 5 is configured to be closable in at least two axial positions 52, 51 in order to generate at least one pouch 53 for storing a pilot or counter liquid sample, as shown in Figure 2. Once the liquid sample is introduced in the chamber 6, part of the liquid sample is isolated within the inlet conduit 5 between the at least two axial positions 52,51. Once the liquid sample is isolated the inlet conduit 5 is closed in such axials position 52, 51. Preferably the second axial position 51, the one that is further from the chamber 6, is closed first.

In a preferred embodiment, the inlet conduit 5 is sealable at least in the at least two axial positions 52,51, for instance, using a sealer. In a preferred embodiment, the inlet conduit 5 is sealable by heat, radio frequency, ultrasound or mechanic means, in a way that the chamber 6 and the pouch 53 are hermetically sealed. In a particular embodiment, the inlet conduit 5 is heat sealable, for instance, using a heated pinch sealer, so that the chamber 6 and pouch 53 are hermetically sealed. In a very preferred embodiment, the, inlet conduit 5 is radio frequency sealable.

In a preferred embodiment, the inlet conduit 5 is configured for being coupled to a valve, cannula, a syringe or a stopper.

In a preferred embodiment, the device further comprises an outlet conduit 4 in communication with the interior of the chamber 6, configured for letting the air exit from inside the chamber 6 through a chamber 6 and outlet conduit 4 connection opening (not shown in Figures) when the liquid sample is introduced into the chamber 6. Preferably, the outlet conduit 4 is located in the hollow body 8, more preferably being coupled to the hollow body 8 in a manner to provide a permanent, leak -proof sealed engagement. Once the chamber 6 is filled with the liquid sample, the outlet conduit 4 is closed in order to prevent the exit of the liquid sample or the introduction of external fluids or elements to the chamber 6 during the storage of the device. The outlet conduit 4 preferably is closable as close as possible to the chamber 6 and outlet conduit 4 connection opening 41.

In one particular embodiment, the outlet conduit 4 is configured to be closable by a cap, a plug, a lid or a cover that hermetically seals the outlet conduit 4 so that the chamber 6 is hermetically sealed. In another particular embodiment, the sealing of the outlet conduit 4 is made by conventional methods known by the persons skilled in the art, for example, by means of heat, radiofrecuency, ultrasounds or mechanic sealing, preferably so that the chamber 6 is hermetically sealed. In a particular embodiment, the outlet conduit 4 is heat sealable, for instance, using a heated pinch sealer. In a very preferred embodiment, the, inlet conduit 5 is radio frequency sealable.

In a preferred embodiment the outlet conduit 4 is configured for housing or to be coupled to a valve, a cannula, a stopper, or a filter, preferably a filter permeable to gas but impermeable to the liquid sample being stored within the chamber 6.

In a preferred embodiment, the outlet conduit 4 is located in the hollow body 8 at the same height or higher than the inlet conduit 5. In a preferred embodiment both conduits 4,5 are at the same height near a one distal end of the hollow body 8. This embodiment is useful for the storage of the device in tube or vial racks, wherein the pouch 53 is going to be localized on the rack during the storage of the device.

In one embodiment, the outlet conduit 4 and the inlet conduit 5 are in opposite sides of the hollow body 8. This embodiment is preferred when a filter is housed in the outlet conduit 4.

In another embodiment, the outlet conduit 4 and the inlet conduit 5 are in the same side of the hollow body 8. The advantage of this embodiment is that when the first axial position 52 of the inlet conduit 5 and the outlet conduit 4 are closed by sealing, for example by heat or radio frequency means, as they are in the same side of the hollow body 8, both conduit 4,5 can be sealed at once or with fewer device manoeuvres by the user.

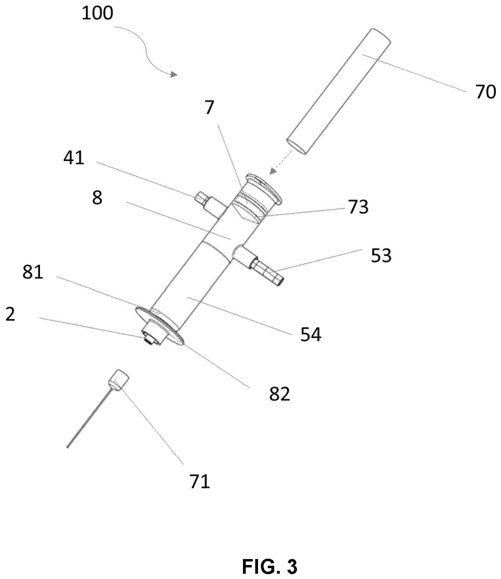

In a preferred embodiment, as it is shown in figures 1 and 3, the inlet conduit 5 and the outlet conduit 4 are located between a first end opening 1 and a second end opening 2 of the hollow body 8.

Regarding the dispensing means, as shown in Figure 1 and 3, in a preferred embodiment the dispensing means comprise:
- a plunger portion 7 contained within the hollow body 8 that is adjacent to the first end opening 1 of the hollow body 8 and that is configured to be movable within the hollow body 8 towards the second end opening 2 of the hollow body 8, being the second end opening 2 in communication with the interior of the chamber 6;
- a plunger rod 70 connectable to the plunger portion 7; and
- optionally, for example when the injection of the liquid sample is needed, a hollow needle 71 connectable to the second end opening 2 of the hollow body 8, optionally being connectable the needle 71 through a luer connecting tube.

In a preferred embodiment, the first end opening 1 and the second end opening 2 of the hollow body 8 are closed with removable attached lids 10, 20 as shown in Figure 1. These lids are attached or adhered to the first and/or second end openings 1,2 until the dispensation of the liquid sample is needed. When the dispensation is needed, the lids 10,20 are removed and the plunger rod 70 and/or the hollow needle 71 can be connected respectively. In another embodiment, the lids 10, 20 that are attached to the first and/or second end openings 1,2 are perforable by the plunger rod 70 and/or the hollow needle 71 respectively.

In a preferred embodiment, the lid 10 that closes the first end opening 1 is a film that is attached or adhered to an external surface of a protrusion 11 of the first end opening 1 that hermetically seals the first end opening 1 and is removable or perforable by the plunger rod 70. This sealing prevents from contamination of the liquid sample contained in the chamber 6 with external elements present in the storage means, for example, from contamination of liquid nitrogen of the freezer until the film is removed or perforated by the plunger rod 70.

In a preferred embodiment, the lid 20 that closes the second end opening 2 is a removably attached cap 20. In a preferred embodiment the attached cap hermetically seals the second opening 2, preventing from contamination with external elements present in the storage means of the liquid sample within the chamber 6. Preferably, the cap 20 is attached to an external protrusion 82 of the hollow body 8 that is above the second end opening 2. This attachment may be for example by gluing or screwing.

In a preferred embodiment, the attached cap 20 comprises a removable internal film or adaptor 21 that seals the second end opening 2. In one embodiment, the adaptor 21 is a plug that is screwed inside the second opening 2. This embodiment prevents from any unwanted opening during the storage, for example, during freezing and thawing of the liquid sample, where the liquid sample and device parts can be subjected to stress changes causing unwanted openings of the cap 20 and/or adaptor 21 during storage.

In a preferred embodiment, during the storage the chamber 6 is hermetically closed or sealed. Thus, depending of the embodiment, the closing of the inlet conduit 5, the outlet conduit 4, the first end opening 1 and second end opening 2 are hermetic, maintaining the liquid sample in an hermetic environment.

Regarding the plunger portion 7, as shown in Figure 1, in a preferred embodiment the shape of the base 73 of the plunger 7 matches the shape of an internal base 81 of the hollow body 8 where the second end opening 2 is located. The shape of the base 73 of the plunger 7 is preferably flat, conical, substantially conical or substantially convex.

Preferably, the parts or elements of the device that are in contact with the liquid sample and/or that will be stored are made of standard materials used in the field of blood banking and long-term storage in cryogenic conditions. Preferably such parts or elements are made of cryogenic conditions resistant material, preferably compatible with biological material. Such parts or elements of the device may comprise different biocompatible polymers or copolymers. In one embodiment, lids 10,20 and the hollow body 8 comprises cyclic olefin copolymer, polypropylene, polyethylene or polycarbonate, preferably rigid cyclic olefin copolymer. In one embodiment the inlet and outlet conduits 5,4 comprise cyclic olefin copolymer, ethylvinylacetate or polyvynilchloride, preferably ethylvinylacetate. In one embodiment, the plunger 7 comprises a thermoplastic polymer (TPE) or silicone.

In a preferred embodiment the device is a syringe.

In a preferred embodiment the hollow body has a tubular shape.

In terms of a capacity, in a preferred embodiment the chamber has a storage maximum capacity of 10ml of liquid sample, preferably between 1 ml and 5 ml.

Regarding the liquid sample, the device is useful for any biological sample or pharmaceutical composition. The liquid sample preferably comprises serum, blood, cells, viruses, bacteria, cell cultures or suspension of cells. In another embodiment, the liquid sample is a vaccine or a immunotherapeutic composition.

A second aspect of the invention is a method for storing and dispensing a liquid sample carried out by means of a device of the invention which comprises the following steps:
- a first step of introducing the liquid sample to be stored into the chamber 6 of the hollow body 8 through the inlet conduit 5,
- a second step of generating at least one pouch 53 for storing a pilot liquid sample in the inlet conduit 5,
- a third step of storing the liquid sample within the chamber 6 and the at least one pouch 53, and
- a fourth step of dispensing the stored liquid sample;
wherein, the step of generating the at least one pouch 53 for storing a pilot liquid sample in the inlet conduit 5 comprises:
- a first step of isolating liquid sample within the inner conduit 5 between a first axial position 52 and a second axial position 51, and,
- a second step of closing the inlet conduit 5 in the first axial position 52 and the second axial position 51 generating the at least one pouch 53.

In a preferred embodiment, the liquid sample is isolated within the inlet conduit 5 by suctioning at least part of said liquid sample from the chamber 6 or at least part of the liquid sample that remains in the inlet conduit 5 next to the chamber 6 and inlet conduit 5 connecting opening.

In a preferred embodiment, one or more additional pilot liquid samples can be generated isolating more liquid sample within the inlet conduit 5 and closing the inlet conduit 5 in a subsequent axial position.

In a preferred embodiment, the second step is a cryopreservation step. Thus, the device with the liquid sample and at least one pouch 53 with the pilot sample is stored in cryopreservation mediums and machines, preferably at ultralow temperatures, below -80°C. Before the dispensing step, the liquid sample is thawed. The method is applicable for standardized cryopreservation methods known by the skilled person in the art, for example, for the cryopreservation methods described in Standardized Cryopreservation of Human Primary Cells. Curr. Protoc. Cell Biol. 64:A.3I.1-A.3I.8. © 2014 by John Wiley & Sons, Inc.

The method can be implemented in any of the embodiments or configurations of the device of the invention, the method thereby comprising the corresponding embodiment or configuration. All the features described for the device which are useful for the method are also considered described for the method.

## Claims

1. Device for storing and dispensing a liquid sample comprising:
- a hollow body (8) comprising a chamber (6) for storing the liquid sample, and
- dispensing means for extracting the liquid sample from the chamber (6),
**characterized in that** the device further comprises an inlet conduit (5) configured for introducing the liquid sample into the chamber (6), the inlet conduit (5) being configured to be closable in at least two axial positions (52, 51) in order to generate at least one pouch (53) in said inlet conduit for storing a pilot liquid sample.

2. Device according to claim 1, wherein the inlet conduit (5) is sealable at least in the at least two axial positions (52, 51).

3. Device according to claim 2, wherein the inlet conduit (5) is heat or radio frequency or ultrasound or mechanically sealable, preferably radio frequency sealable.

4. Device according to any of the preceding claims, wherein the inlet conduit (5) is configured for being coupled to a valve, cannula, stopper and/or syringe.

5. Device according to any of the preceding claims, wherein the device further comprises an outlet conduit (4) configured for letting the air exit from inside the chamber (6) and being configured to be closable.

6. Device according to claim 5, wherein the outlet conduit (4) is heat or radio frequency or ultrasound or mechanically sealable, preferably radio frequency sealable.

7. Device according to claim 5 or 6, wherein the outlet conduit (4) is configured for housing a filter (9).

8. Device according to any of claims 5 to 7, wherein the outlet conduit (4) is located in the hollow body (8) at the same height or higher than the inlet conduit (5).

9. Device according to any of the preceding claims, wherein the dispensing means comprise:
- a plunger portion (7) contained within the hollow body (8) that is adjacent to a first end opening (1) of the hollow body (8) and that is configured to be movable within the hollow body (8) towards a second end opening (2) of the hollow body (8);
- a plunger rod connectable to the plunger portion (7); and
- optionally a hollow needle connectable to the second end opening (2) of the hollow body (8).

10. Device according to claim 9, wherein the first end opening (1) and the second end opening (2) of the hollow body (8) are closed with removably attached or perforable lids (10, 20).

11. Device according to claim 10, wherein the lid (10) that closes the first end opening (1) is a film that is attached or adhered to an external surface of a protrusion (11) of the first end opening (1).

12. Device according to any of claims 9 to 11, wherein the lid (20) that closes the second end opening (2) is a removably attached cap, preferably attached to an external protrusion (82) of the hollow body (8) above the second end opening (2).

13. Device according to claim 12, wherein the removably attached cap comprises an internal film or adaptor (21) that removably seals the second end opening (2).

14. Device according to any of the preceding claims, wherein the device is made of material resistant to cryo storage conditions.

15. Device according to any of the preceding claims, wherein the liquid sample is serum, blood, cells, viruses, bacteria, cell cultures, suspension of cells, vaccine, immunotherapeutic composition or a pharmaceutical composition.

16. Method for storing and dispensing a liquid sample carried out by means of a device according to any of claims 1 to 15, which comprises the following steps:
- a first step of introducing the liquid sample to be stored into the chamber (6) of the hollow body (8) through the inlet conduit (5),
- a second step of generating at least one pouch (53) for storing a pilot liquid sample in the inlet conduit (5),
- a third step of storing the liquid sample within the chamber (6) and the at least one pouch (53),and
- a fourth step of dispensing the stored liquid sample;
wherein, the step of generating the at least one pouch (53) for storing a pilot liquid sample in the inlet conduit (5) comprises:
- a first step of isolating liquid sample within the inner conduit (5) between a first axial position (52) and a second axial position (51), and,
- a second step of closing the inlet conduit (5) in the first axial position (52) and the second axial position (51) generating the at least one pouch (53).

17. Method according to claim 16, wherein the liquid sample is isolated within the inlet conduit (5) by suctioning at least part of said liquid sample from the chamber (6).

18. Method according to claim 16 or 17, wherein the liquid sample is isolated within the inlet conduit (5) by suctioning at least part of the liquid sample that remains in the inlet conduit (5) next to the chamber (6) and the inlet conduit (5) connection opening.

19. Method according to claim 16 to 18, wherein one or more additional pilot liquid samples are generated introducing more liquid sample within the inlet conduit (5) and closing the inlet conduit (5) in a subsequent axial position.

20. Method according to any of claims 16 to 19, wherein the third step is a cryopreservation step.
